# EUROPEAN PATENT SPECIFICATION

(11) **EP 2 668 440 B1**
(45) Date of publication and mention of the grant of the patent: **20.08.2014**
(21) Application number: 12703566.5
(22) Date of filing: 24.01.2012
(51) Int. Cl.: F17C 1/16

(54) **GAS ACCUMULATOR**
GASSPEICHER
ACCUMULATEUR DE GAZ

(30) Priority: 27.01.2011 GB 201101389
(43) Date of publication of application: 04.12.2013
(73) Proprietor: Base Structures Limited, Bristol BS2 0UY (GB)
(72) Inventor: MORRIS, Jon, Bristol BS2 0UY (GB)
(74) Representative: Howell, Matthew Dafydd
(86) International application number: PCT/GB2012/050149
(87) International publication number: WO 2012/101434

(56) References cited:
- EP-A2- 1 338 843

## Description

### Field of Invention

The present invention relates to membrane gas accumulators that can be used to collect and store bio-gas.

### Background to the Invention

Membrane gas accumulators are commonly used to collect and store bio-gas released by the anaerobic respiration of various bacteria within sewage or other rotting organic matter

Some existing gas accumulators, such as the one disclosed in EP1338843, consist of two membranes. The first of these is an inner gas-impermeable membrane which sits on the ground or on a support surface such as a concrete slab and forms at least part of a gas accumulation chamber. The membrane must therefore either be anchored to the ground in a gas-impermeable manner, or may form the complete gas accumulation chamber.

The second membrane is an outer gas-impermeable membrane. This membrane at least partially encloses the first membrane, and protects it from external forces. The outer membrane must therefore be strong and durable enough to resist forces caused by wind and other adverse weather conditions. In the event of failure or decompression of the outer membrane, all external forces will be transferred to the inner membrane, and therefore the inner membrane must also be structurally able to withstand external forces.

A pressurisation chamber is formed between the two membranes. The pressurisation chamber can be inflated or deflated to regulate the flow of gas into and out of the gas accumulation chamber. For example, the pressurisation chamber can be inflated to cause gas in the gas accumulation chamber to be released at a constant rate once the gas accumulation chamber is full. The pressurisation chamber has a mechanism for the introduction and extraction of an auxiliary gas, in order to maintain and adjust the In much of the prior art, edges of the membranes are attached to a base support surface to secure the membranes to the surface. The attachment formations used may have to be air and gas tight, and so are laborious and expensive to install.

Existing membrane gas accumulators have both the first and second membranes anchored to a base support surface, typically using staples or clamp plates which engage with ropes, rods or directly with the fabric, clamping via a keder or bolt rope edge or similar. This means that installing the accumulator is time-consuming, as potentially two rows of staples or bolted clamp plates must be inserted and attached to the membranes around the entire circumference of the accumulator.

Existing gas accumulators can also be expensive. Both the first and second membranes must be of a material that is strong enough to withstand the stresses of forces such as wind and bad weather. Putting the gas accumulation chamber under such stresses may also increase the risk of damage to the membrane, which may result in gas leaks. More material is also needed to create the attachments on both membranes.

A variety of existing membrane gas accumulators has three membranes. The third membrane is attached in a gas-impermeable manner to at least the second membrane, and helps to define the pressurisation chamber. The pressurisation chamber is therefore not defined by the first and second membranes, but by the second and third membranes.

This creates a layer of non-pressurised gas between the gas accumulation chamber and the pressurisation chamber. In other gas accumulators, any gas leak from the gas accumulation chamber would enter the pressurisation chamber, which would then contain a dangerous and potentially explosive mix of gases. In three-membrane accumulators, any gas escaping from the gas accumulation chamber enters the layer of air between the first and the third membranes, and can thereby escape the gas accumulator. Any gas leak can also be noticed quickly and easily, as a leak from the gas accumulation chamber cannot be compensated for by the pressurisation chamber, as can happen in models with two membranes if the two chambers are connected by the leak.

Different means for monitoring the volume of the gas accumulation chamber are known in the art. In an existing embodiment, this monitoring means consists of an element that is suspended in the pressurisation chamber and partially rests on the third membrane. As the gas accumulation chamber fills, the third membrane takes some of the weight of the element. The change in tension due to the weight of the portion of the element that remains suspended is detected by a device outside the gas accumulator.

Such a monitoring system does not connect the two membranes, and thus does not place additional stress on the membranes, thereby reducing the probability of damage. However, there is still some possibility of damage by the element to the first membrane. This monitoring system would also not work unless the second membrane can form a dome shape without the first membrane being full and providing pressure to hold the dome up. This method is therefore not suitable for all designs of gas accumulator.

Known gas accumulators also have a problem with damage from rodents. Rodents such as mice and rats eat the PVC that the membranes are often made of, thereby causing damage and potential leaks.

### Summary of the Invention

According to a first aspect of the invention there is provided a gas accumulator for the storage of bio-gas, comprising: a first gas-impermeable membrane, which at least partially defines a gas accumulation chamber; a second gas-impermeable membrane; a third gas-impermeable membrane, which is attached to the second membrane by means of a gas-impermeable seal such that the second and third membranes together define a gas-impermeable pressurisation chamber; means for pressurising said pressurisation chamber by introduction of an auxiliary gas; and means for anchoring the gas accumulator to a support surface; characterised in that the anchoring means is configured to anchor only the second membrane to the support surface.

In the gas accumulator of the present invention the anchoring means only engages with the second membrane. This reduces the time needed to install the gas accumulator, compared to existing gas accumulators in which both membranes are anchored. By anchoring only the second membrane the first membrane need not be load bearing, and therefore can be made of a lighter and possibly cheaper material than in the prior art. Additionally less material may be needed to anchor only the second membrane, which may further reduce the cost of the gas accumulator in comparison to prior art systems.

The anchoring means may comprise a staple which engages with a pole running through a fabric pocket attached to the second membrane, the staple having free ends which are received in the support surface. Such anchoring means are cost effective and do not take a long time to install.

It is to be appreciated, however, that other anchoring means may be used, for example a series of D rings fixed to an outer surface of the second membrane via catenary webbing which itself is stitched to the third membrane to engage via a hook with an anchor ring that is secured in the base support surface.

A base of the gas accumulation chamber may comprise the first membrane. This creates a gas-impermeable gas accumulation chamber, without the anchoring means having to provide a gas-impermeable seal. The anchoring means can therefore be mechanical, and can be cheap and fast to install.

The gas accumulator may provide a layer of non-pressurised gas between the pressurisation chamber and the gas accumulation chamber.

All three membranes are flexible and gas-impermeable, such that if gas escapes from the gas accumulation chamber, it enters this layer of non-pressurised gas rather than entering the pressurisation chamber, which is potentially dangerous.

The pressurisation chamber may be provided with a valve for the introduction and extraction of the auxiliary gas. This allows control of the rate of gas being expelled from the gas accumulation chamber when it is full.

The auxiliary gas preferably is air, as air is cheap and readily available, though other gases can be used.

The gas accumulation chamber may comprise means for extracting and introducing gas, for example by means of a valve mounted in a flange on the first membrane.

The gas accumulator may further comprise means for detecting the volume of gas in the gas accumulation chamber.

The means for detecting the volume of gas in the gas accumulation chamber may comprise an ultra-sound level detector. This allows the volume of the gas accumulation chamber to be monitored without connecting the first and third membranes, thereby preventing stress on the first membrane and the possibility of damage.

A flange may be provided at an apex of the second membrane of the gas accumulator in which the ultrasound level detector can be received.

The first membrane may be made of a material which is lighter in weight than that of the second and third membranes.

For example, the first membrane, which forms the non-structural gas accumulation chamber, may be made of laminated and calendered PVC and the second and third membranes, which are structural, may be made of Polyester reinforced PVC.

The second membrane may extend beyond the point at which the fabric pockets are attached to the second membrane.

In use of the gas accumulator, an edge of the second membrane may be disposed beneath the gas accumulation chamber. This impedes access to the first membrane to rodents, thereby protecting the first membrane from damage and potential gas leaks.

A condensate drain may be provided towards the base of the gas accumulation chamber. This allows excess liquid to be drained from the gas accumulation chamber. The first membrane may be covered by a material having a lubricating and anti-static characteristic. This helps to prevent damage to the membrane due to friction when it contacts the third membrane.

The material may be, polyurethane coated glass fibre fabric for example.

The gas accumulator may be generally dome shaped when full.

Alternatively, the gas accumulator may be generally cylindrical when full.

### Brief description of the drawings

Embodiments of the invention will now be described, by way of example only, with reference to the accompanying drawings, of which:
Figure 1 is a cross-sectional view of a gas accumulator when the gas accumulation chamber is full;
Figures 2 is a cross-sectional view of a section of a base edge of the gas accumulator; and
Figure 3 is a cross-sectional view of a section of a base edge of the gas accumulator showing alternative anchoring means.

### Description

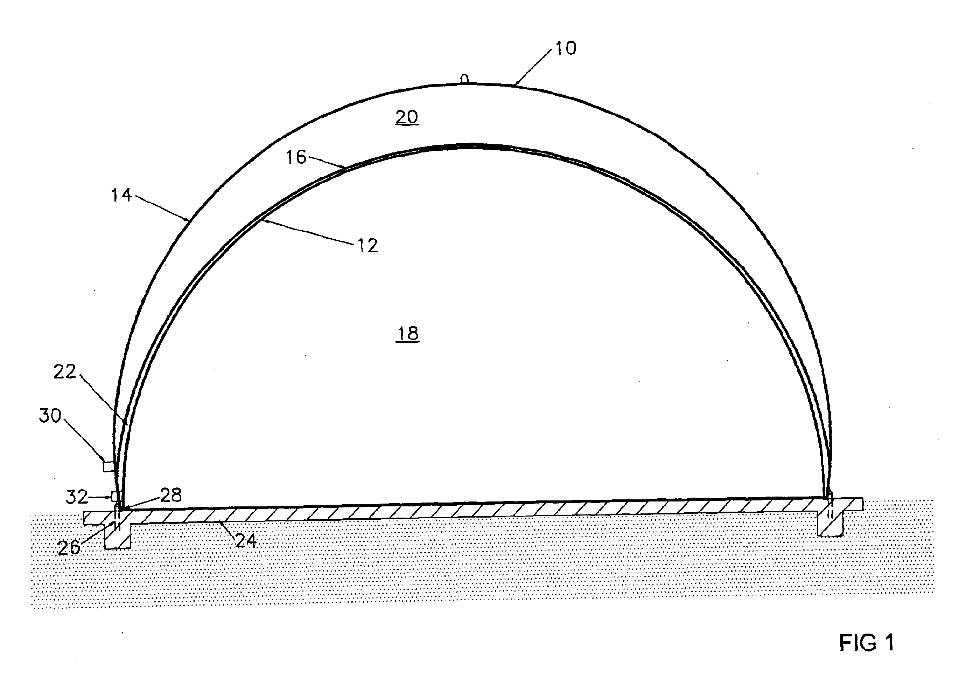

Referring first to Figure 1, a gas accumulator is shown generally at 10, and includes a first, flexible and gas-impermeable membrane 12, a second, outer, flexible and gas-impermeable membrane 14 and a third, intermediate flexible and gas-impermeable membrane 16.

The first membrane 12 has a base or floor portion and a roof portion and defines a gas accumulation chamber 18 which, when filled with gas, assumes a generally dome-like shape.

The second membrane 14 fully encloses the first membrane 12, also forming a generally dome-like shape.

The third membrane 16 is attached to the second membrane 14 by means of a gas-impermeable seal and, with the second membrane, defines a pressurisation chamber 20.

Between the first membrane 12 and the third membrane 16 is a layer of non-pressurised gas 22. Any gas escaping from the gas accumulation chamber 18 enters this layer 22 and can escape the gas accumulator 10.

As all three of the membranes 12, 14, 16 are flexible and gas-impermeable, if gas escapes from the gas accumulation chamber 18, it enters this layer of non-pressurised gas rather than entering the pressurisation chamber 20, which is potentially dangerous. This arrangement also allows any leaks to be detected quickly, as in the event of a leak from the gas accumulation chamber 18 the gas accumulation chamber 18 will deflate, causing the shape of the gas accumulator 10, or at least the shape of the gas accumulation chamber 18, to change.

The first and third membranes 12, 16, or sections thereof may be coated or otherwise provided with a low friction and anti-static material or with a material having a lubricating characteristic, for example, polyurethane coated glass fibre. This helps to prevent damage to both membranes 12, 16 due to friction when the coated sections are in contact with one another.

The second membrane 14 is anchored to the base support surface 24 by anchoring means 26, as is described in more detail below.

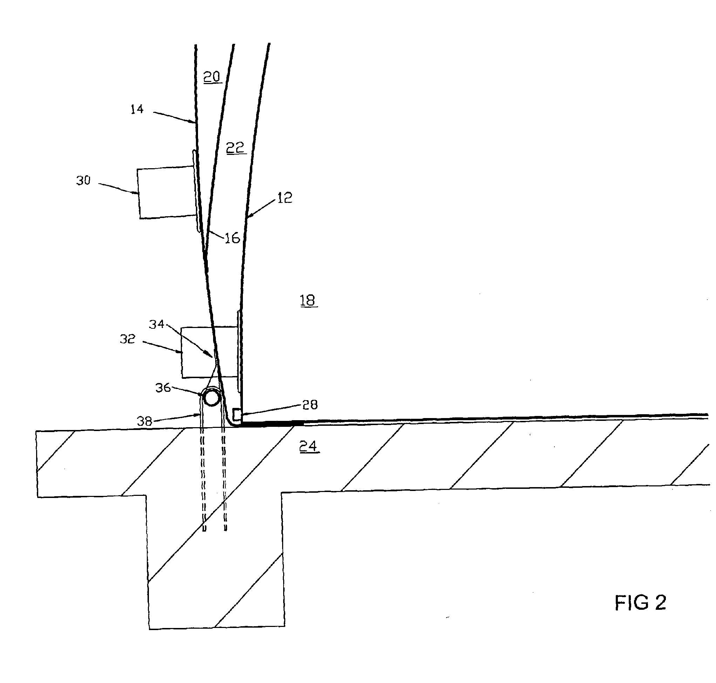

Figure 2 is a view of a section of a base edge of the gas accumulator 10, showing how the gas accumulator 10 is anchored to the ground or to another support surface. A fabric pocket 34 is attached to the outside of the second membrane 14, and a pole 36 runs through the fabric pocket 34. This pole runs around the circumference of the gas accumulator 10 through additional fabric pockets 34 disposed around a perimeter of the second membrane 14 of the gas accumulator 10, and is retained by staples 38 whose legs are embedded in the ground or the support surface, thereby anchoring the second membrane 14 to the base support surface 24.

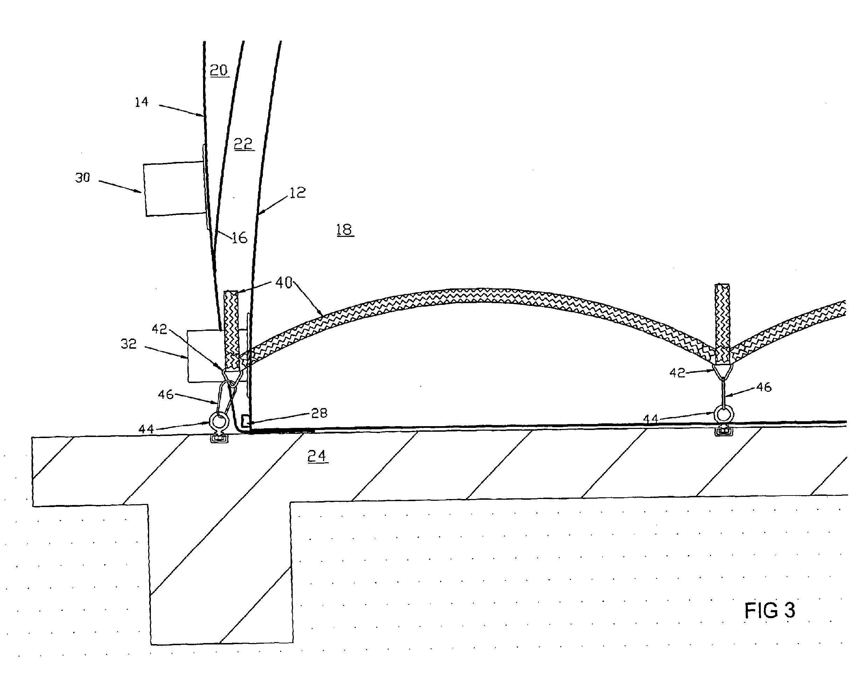

Figure 3 is a view of a section of a base edge of an alternative embodiment of the gas accumulator 10. In the embodiment shown in Figure 3, a webbing belt 40 is attached to the outside of the second membrane 14. The webbing belt 40 runs around the circumference of the gas accumulator 10 generally describing a continuous catenary edge and incorporating, at intervals (which may be, for example, 1.5 metres), D rings 42. The D rings 42 are attached to anchor rings 44 which are embedded in the ground or the support surface 24, via clips 46, thereby anchoring the second membrane 14 to the base support surface 24.

It will be appreciated from the description above that only the second membrane 14 is anchored to the ground or the support surface, and thus only the second membrane 14 must be load bearing. As the second membrane 14 completely encloses the first membrane 12, the second membrane 14 provides support and protection for the first membrane 12.

As the first membrane 12 need not be load bearing, it can be made of a lighter weight material than the second and third membranes 14, 16. For example, the first membrane 12, which forms the non-structural accumulation chamber, can be made of laminated and calendered PVC, and the second and third membranes 14, 16, which are structural, can be made of Polyester reinforced PVC. The material of the first membrane 12 may be less expensive than that of the second and third membranes 14,16, due to its lighter weight and lower specification.

As is shown in Figure 2, the second membrane 14 extends beyond the point at which the fabric pockets 34 or webbing 40 are attached to the second membrane 14, thereby defining an edge of the second membrane 14, which in use of the gas accumulator 10 can be disposed beneath the gas accumulation chamber 18, as shown in Figures 2 and 3. This helps to prevent damage to the first membrane 12 by rodents and other animals, thereby helping to prevent leaks from the gas accumulation chamber 18.

A condensate drain 28 is provided towards a base of the first membrane 12 and allows excess liquid to be drained from the gas accumulation chamber 18.

Auxiliary gas such as air is introduced into and extracted from the pressurisation chamber 20 through a valve 30, which is provided in part of the second membrane 14.

Gas is introduced and extracted from the gas accumulation chamber 18 through a valve in a flange 32, which is part of the first membrane 12.

A flange (not shown) may be provided towards an apex of the outer second membrane 14, and may include a recess or other receiving formation for receiving means for detecting the volume of gas in the gas accumulation chamber 18. In one example, the means for detecting the volume of gas in the gas accumulation chamber is an ultrasonic level detector, which can be used to monitor the volume of gas in the gas accumulation chamber 18 without connecting the first and third membranes 12, 16, thereby preventing stress on the first membrane 12 and reducing the possibility of damage to the first or third membranes 12, 16.

In use of the gas accumulator 10, gas to be stored is deposited in the gas accumulation chamber 18 by means of the valve 32, until the gas accumulation chamber 18 is full. When gas is to be released from the gas accumulation chamber 18, the valve 32 in the gas accumulation chamber 18 is opened and air or another auxiliary gas is pumped into the pressurisation chamber 20 via valve 30, thereby pressurising the gas accumulation chamber 18 and causing gas to escape via the valve 32. Air is preferred as the auxiliary gas as it is freely available, but it will be understood that other gases may be used if necessary or desired.

The gas accumulation chamber 18 of the gas accumulator 10 described above and shown in Figures 1 and 2 is defined by the floor and roof portions of the first membrane 12. In other words, the first membrane 12 of the gas accumulator 10 shown above forms a gas impermeable dome for storing gas.

Although the gas accumulator 10 described above and shown in Figures 1, 2 and 3 is generally dome-shaped, it will be appreciated by those skilled in the art that the principles of the present invention are equally applicable to other shapes of gas accumulators, such as, for example, cylindrical or pyramid-shaped gas accumulators.

## Claims

1. A gas accumulator (10) for the storage of bio-gas, comprising:
- a first gas-impermeable membrane (12), which at least partially defines a gas accumulation chamber (18);
- a second gas-impermeable membrane (14);
- a third gas-impermeable membrane (16), which is attached to the second membrane (14) by means of a gas-impermeable seal such that the second and third membranes (14, 16) together defme a gas-impermeable pressurisation chamber (20);
- means for pressurising said pressurisation chamber by introduction of an auxiliary gas; and
- means (38) for anchoring the gas accumulator (10) to a support surface, **characterised in that** the anchoring means (38) is configured to anchor only the second membrane (14) to the support surface.

2. A gas accumulator (10) according to claim 1 wherein the anchoring means (38) comprises a staple which engages with a pole (36) running through a fabric pocket (34) attached to the second membrane (14), the staple (38) having free ends which are received in the support surface.

3. A gas accumulator (10) according to claim 1 or 2 wherein a base of the gas accumulation chamber (18) comprises the first membrane (12).

4. A gas accumulator (10) according to any preceding claim wherein between the pressurisation chamber (20) and the gas accumulation chamber (18) is a layer of non-pressurised air.

5. A gas accumulator (10) according to any preceding claim wherein the pressurisation chamber (20) is provided with a valve (30) for the introduction and extraction of the auxiliary gas.

6. A gas accumulator (10) according to any preceding claim wherein the gas accumulation chamber (18) further comprises means for extracting and introducing gas.

7. A gas accumulator (10) according to claim 6 wherein the means for extracting and introducing gas comprises a valve mounted in a flange (30) on the first membrane (12).

8. A gas accumulator (10) according to any preceding claim further comprising means for detecting the volume of the gas in the gas accumulation chamber (18).

9. A gas accumulator (10) according to claim 8 wherein the means for detecting the volume of the gas in the gas accumulation chamber (18) comprises an ultrasound level detector.

10. A gas accumulator (10) according to any preceding claim wherein the first membrane (12) comprises a fabric that is lighter in weight than the second membrane (14).

11. A gas accumulator (10) according to claim 8 wherein the fabric of the first membrane (12) is laminated and calendered PVC and the fabric of the second membrane (14) is polyester reinforced PVC.

12. A gas accumulator according to claim 2 wherein the second membrane (14) extends beyond a point at which the fabric pockets (34) are attached to the second membrane (14).

13. A gas accumulator (10) according to claim 12 wherein, in use, an edge of the second membrane (14) is disposed beneath the gas accumulation chamber (18).

14. A gas accumulator (10) according to any preceding claim wherein the first membrane (12) includes a condensate drain.

15. A gas accumulator (10) according to any preceding claim wherein sections of the first membrane (12) and the third membrane (16) are coated with a material having an anti-static and lubricating characteristic.

## Patentansprüche

1. Gasspeicher (10) zum Speichern von Biogas, mit
- einer ersten gasundurchlässigen Membran (12), welche zumindest teilweise eine Gasspeicherkammer (18) bildet,
- einer zweiten gasundurchlässigen Membran (14),
- einer dritten gasundurchlässigen Membran (16), welche an der zweiten Membran (14) mittels einer gasundurchlässigen Dichtung derart befestigt ist, dass die zweite und die dritte Membran (14, 16) zusammen eine gasundurchlässige Druckbeaufschlagungskammer (20) bilden,
- einer Einrichtung zum Unterdrucksetzen der Druckbeaufschlagungskammer durch Einbringen eines Hilfsgases; und
- einer Einrichtung (38) zur Verankerung des Gasspeichers (10) an einer Auflagefläche,
**dadurch gekennzeichnet, dass** die Verankerungseinrichtung (38) derart ausgebildet ist, dass sie nur die zweite Membran (14) an der Auflagefläche verankert.

2. Gasspeicher (10) nach Anspruch 1, bei welcher die Verankerungseinrichtung (38) eine Klammer aufweist, welche mit einer Stange (36) in Eingriff steht, die durch eine an der zweiten Membran (14) befestigte Stofftasche (34) verläuft, wobei freie Enden der Klammer (38) von der Auflagefläche aufgenommen sind.

3. Gasspeicher (10) nach Anspruch 1 oder 2, bei welchem die erste Membran (12) die Unterseite der Gasspeicherkammer (18) bildet.

4. Gasspeicher (10) nach einem der vorstehenden Ansprüche, bei welcher sich zwischen der Druckbeaufschlagungskammer (20) und der Gasspeicherkammer (18) eine Schicht unkomprimierter Luft befindet.

5. Gasspeicher (10) nach einem der vorstehenden Ansprüche, bei welcher die Druckbeaufschlagungskammer (20) mit einem Ventil (30) zum Einführen und Entnehmen des Hilfsgases versehen ist.

6. Gasspeicher (10) nach einem der vorstehenden Ansprüche, bei welchem die Gasspeicherkammer (18) weiterhin eine Einführung zum Entnehmen und Einbringen von Gas aufweist.

7. Gasspeicher (10) nach Anspruch 6, bei welchem die Einrichtung zum Entnehmen und Einbringen von Gas ein in einem Flansch (30) der ersten Membran (12) angeordnetes Ventil umfasst.

8. Gasspeicher (10) nach einem der vorstehenden Ansprüche, ferner mit einer Einrichtung zum Ermitteln des Gasvolumens in der Gasspeicherkammer (18).

9. Gasspeicher (10) nach Anspruch 8, bei welchem die Einrichtung zum Ermitteln des Gasvolumens in der Gasspeicherkammer (18) einen Ultraschallpegeldetektor aufweist.

10. Gasspeicher (10) nach einem der vorstehenden Ansprüche, bei welchem die erste Membran (12) ein Gewebe aufweist, welches leichter im Gewicht als die zweite Membran (14) ist.

11. Gasspeicher (10) nach Anspruch 8, bei welchem der Stoff der ersten Membran (12) ein laminiertes und kalandriertes PVC-Material und das Gewebe der zweiten Membran (14) ein polyesterversteiftes PVC-Material ist.

12. Gasspeicher nach Anspruch 2, bei welchem die zweite Membran (14) über einen Punkt hinaus reicht, an welchem die Stofftaschen (34) an der zweiten Membran (14) befestigt sind.

13. Gasspeicher (10) nach Anspruch 12, bei welchem im Gebrauch eine Kante der zweiten Membran (14) sich unterhalb der Gasspeicherkammer (18) befindet.

14. Gasspeicher (10) nach einem der vorstehenden Ansprüche, bei welchem die erste Membran (12) einen Kondensatabschluss enthält.

15. Gasspeicher (10) nach einem der vorstehenden Ansprüche, bei welchem Abschnitte der ersten Membran (12) und der dritten Membran (16) mit einem Material beschichtet sind, welches Antistatik- und Schmiereigenschaften hat.

## Revendications

1. Accumulateur de gaz (10) pour le stockage de biogaz, comprenant :
- une première membrane imperméable aux gaz (12) qui définit au moins partiellement une chambre d'accumulation de gaz (18) ;
- une deuxième membrane imperméable aux gaz (14) ;
- une troisième membrane imperméable aux gaz (16), qui est attachée à la deuxième membrane (14) au moyen d'un joint imperméable aux gaz de façon que les deuxième et troisième membranes (14, 16) définissent ensemble une chambre de pressurisation imperméable aux gaz (20) ;
- des moyens pour pressuriser ladite chambre de pressurisation par introduction d'un gaz auxiliaire ; et
- des moyens (38) pour ancrer l'accumulateur de gaz (10) à une surface de support,
**caractérisé en ce que** les moyens d'ancrage (38) sont configurés pour ancrer uniquement la deuxième membrane (14) à la surface de support.

2. Accumulateur de gaz (10) selon la revendication 1, dans lequel les moyens d'ancrage (38) comprennent une agrafe qui s'engage avec une tringle (36) s'étendant sur une poche en étoffe (34) attachée à la deuxième membrane (14), l'agrafe (38) présentant des extrémités libres qui sont reçues dans la surface de support.

3. Accumulateur de gaz (10) selon la revendication 1 ou 2, dans lequel une base de la chambre d'accumulation de gaz (18) comprend la première membrane (12).

4. Accumulateur de gaz (10) selon l'une quelconque des revendications précédentes, dans lequel il y a une couche d'air non pressurisé entre la chambre de pressurisation (20) et la chambre d'accumulation de gaz (18).

5. Accumulateur de gaz (10) selon l'une quelconque des revendications précédentes, dans lequel la chambre de pressurisation (20) est dotée d'une valve (30) pour l'introduction et l'extraction du gaz auxiliaire.

6. Accumulateur de gaz (10) selon l'une quelconque des revendications précédentes, dans lequel la chambre d'accumulation de gaz (18) comprend en outre des moyens pour extraire et introduire un gaz.

7. Accumulateur de gaz (10) selon la revendication 6, dans lequel les moyens pour extraire et introduire un gaz comprennent une valve montée dans un rebord (30) sur la première membrane (12).

8. Accumulateur de gaz (10) selon l'une quelconque des revendications précédentes, comprenant en outre des moyens pour détecter le volume du gaz dans la chambre d'accumulation de gaz (18).

9. Accumulateur de gaz (10) selon la revendication 8, dans lequel les moyens pour détecter le volume du gaz dans la chambre d'accumulation de gaz (18) comprennent un détecteur de niveau ultrasonique.

10. Accumulateur de gaz (10) selon l'une quelconque des revendications précédentes, dans lequel la première membrane (12) comprend une étoffe dont le poids est inférieur à celui de la deuxième membrane (14).

11. Accumulateur de gaz (10) selon la revendication 8, dans lequel l'étoffe de la première membrane (12) est en PVC stratifié et calandré, et l'étoffe de la deuxième membrane (14) est en PVC renforcé par du polyester.

12. Accumulateur de gaz (10) selon la revendication 2, dans lequel la deuxième membrane (14) s'étend au-delà d'un point au niveau duquel les poches d'étoffe (34) sont attachées à la deuxième membrane (14).

13. Accumulateur de gaz (10) selon la revendication 12, dans lequel, lors de l'utilisation, un bord de la deuxième membrane (14) est disposé sous la chambre d'accumulation de gaz (18).

14. Accumulateur de gaz (10) selon l'une quelconque des revendications précédentes, dans lequel la première membrane (12) comprend un drain de condensat.

15. Accumulateur de gaz (10) selon l'une quelconque des revendications précédentes, dans lequel des sections de la première membrane (12) et de la troisième membrane (16) sont revêtues d'un matériau présentant une caractéristique antistatique et lubrifiante.
